(19) Europäisches Patentamt
European Patent Office
Office européen des brevets

(11) Veröffentlichungsnummer: **0 290 991 B1**

# (12) EUROPÄISCHE PATENTSCHRIFT

(45) Veröffentlichungstag der Patentschrift: **29.07.92**

(51) Int. Cl.5: **C07D 231/12**

(21) Anmeldenummer: **88107407.4**

(22) Anmeldetag: **07.05.88**

## (54) Verfahren zur Herstellung von Pyrazolen.

(30) Priorität: **15.05.87 DE 3716293**

(43) Veröffentlichungstag der Anmeldung:
**17.11.88 Patentblatt 88/46**

(45) Bekanntmachung des Hinweises auf die Patenterteilung:
**29.07.92 Patentblatt 92/31**

(84) Benannte Vertragsstaaten:
**CH DE FR GB IT LI**

(56) Entgegenhaltungen:
**EP-A- 0 045 394**
**DE-A- 3 209 148**

**CHEMICAL ABSTRACTS, Band 95, Nr. 23, 7. Dezember 1981, Columbus, Ohio, US; DARBINYAN, E.G.; MATSOYAN, M.S:; SAAKYAN, A.A.; DZHRAGATSPANYAN, M.A.; MATSOYAN, S.G.: "3-Methylpyrazole" Spalte 2, Zusammenfassung-Nr. 203 946x**

(73) Patentinhaber: **BASF Aktiengesellschaft**
**Carl-Bosch-Strasse 38**
**W-6700 Ludwigshafen(DE)**

(72) Erfinder: **Dockner, Toni, Dr.**
**Grossgasse 6**
**W-6701 Meckenheim(DE)**
Erfinder: **Krug, Herbert**
**Mussbacher Strasse 49**
**W-6700 Ludwigshafen(DE)**
Erfinder: **Rotermund, Gerhard W., Dr.**
**Gluckstrasse 5**
**W-6800 Mannheim 1(DE)**
Erfinder: **Weyrauch, Volker, Dr.**
**Weinstrasse 53**
**W-6730 Neustadt(DE)**

**EP 0 290 991 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von Pyrazolen durch Dehydrierung von Pyrazolinen bei Temperaturen oberhalb 50 ° C in Gegenwart von Schwefel.

Aus Houben/Weyl, Methoden der org. Chemie, Band IV/1b, S. 975-985, ist ein Verfahren zur Dehydrierung von partiell gesättigten N-Heterocyclen mit Nitroaromaten in Abwesenheit von Schwefel bekannt. Dieses Verfahren versagt im Falle von Pyrazolen.

Bislang sind insbesondere für die Dehydrierung des unsubstituierten Pyrazolins nur Wege beschrieben worden, die vor allem unter dem Gesichtspunkt präparativer Durchführbarkeit nicht befriedigen konnten. So lassen sich Pyrazole durch Oxidation von 2-Pyrazolinen herstellen, wobei man als Oxidationsmittel z.B. Chlor und Hypochlorite (EP-PS 48 373) oder Kaliumpermanganat, Salpetersäure, Luft, Bleitetraacetat oder Chromtrioxid (Weißberger, The Chemistry of Heterocyclic Compounds, Band 22, Seiten 41 bis 49 (1967)) verwendet.

Grandberg und Kost (J. Gen. Chem. 28, 3102-3105 (1958), entdeckten in der Umsetzung von elementarem Schwefel oder Selen mit Pyrazolinen eine Dehydriermethode, die bei zahlreichen Alkylpyrazolinen (Reaktionstemperatur 150 bis 205 ° C) in stark exothermer und offenbar auch im kleinen Labormaßstab schlecht kontrollierbarer Reaktion, Ausbeuten an Alkylpyrazolen von 65 bis 96 % liefert.

Die beschriebene Methodik ist für die präparative Darstellung größerer Mengen (Maßstab der Beispiele 0,2 Mol) ungeeignet und keinesfalls auf technische Maßstäbe übertragbar, da die Ausbeuten des Verfahrens bei größeren Ansätzen stark zurückgehen.

In der DE-OS 30 29 160 (EP-PS 45 394) wird ein Verfahren beschrieben, bei dem Schwefel bzw. Selen in Lösungsmitteln zur Dehydrierung von Pyrazolinen bei Temperaturen oberhalb 50 ° C eingesetzt werden. Doch muß auch bei diesem Prozeß mit mindestens stöchiometrischen Mengen Schwefel gearbeitet werden, so daß eine umfangreiche Entsorgung oder Rückführung von Schwefelwasserstoff notwendig wird.

Es bestand nun die Aufgabe, ein Verfahren zur Dehydrierung von Pyrazolinen zu finden, bei dem die geschilderten Nachteile, insbesondere der Anfall von großen Mengen Schwefelwasserstoff, vermieden wird.

Demgemäß wurde ein Verfahren zur Herstellung von Pyrazolen durch Dehydrierung von Pyrazolinen bei Temperaturen oberhalb 50 ° C bis zu 150 ° C in Gegenwart von Schwefel gefunden, das dadurch gekennzeichnet ist, daß man Pyrazoline mit Nitroaromaten in Gegenwart katalytischer Mengen Schwefel umsetzt.

Die Umsetzung läßt sich bei Verwendung von Nitrobenzol als Oxidationsmittel durch folgende Reaktionsgleichung beschreiben:

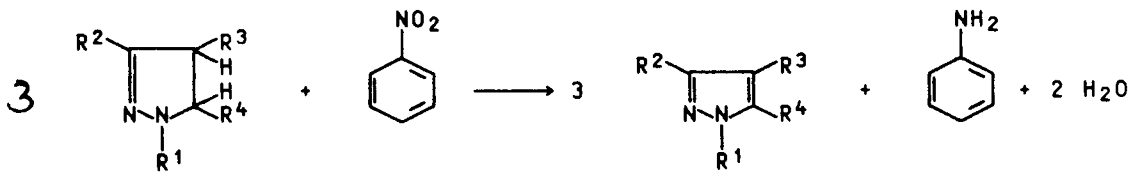

Die Reste $R^1$ bis $R^4$ können dabei gleich oder verschieden sein und jeweils ein Wasserstoffatom, einen aliphatischen, araliphatischen oder aromatischen Rest bedeuten. $R^2$ kann auch ein Halogenatom, der Rest C≡N oder der Rest $-OR^5$ sein, wobei $R^5$ einen aliphatischen, araliphatischedn oder aromatischen Rest bedeutet,
$R^1$ kann auch für den Rest

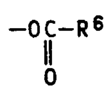

stehen, wobei $R^6$ die für $R^5$ genannte Bedeutung hat. Vorzugsweise sind die Reste $R^1$ bis $R^4$ Wasserstoff.

Aliphatische Reste sind z.B. Alkyl- oder Alkenylreste mit 1 bis 20, insbesondere 1 bis 8 Kohlenstoffatomen. Araliphatische Reste sind z.B. Alkylaryl- oder Arylalkylreste mit 7 bis 12 Kohlenstoffatomen. Aromatische Reste sind z.B. Phenyl- oder Naphthylreste oder heteroaromatische Reste, insbesondere solche mit 5 oder 6 Ringgliedern und 1 oder 2 Heteroatomen, wie Stickstoff, Sauerstoff oder Schwefel. Die genannten

Reste können noch unter den Reaktionsbedingungen inerte Substituenten tragen, z.B. Alkyl- oder Alkoxireste, Halogen, Cyano, Amino-, Carboxyl- oder Sulfogruppen.

Die einzusetzenden Pyrazoline können beispielsweise sein: Pyrazolin, 3-Methyl-, 4-Methyl-, 3,4-Dimethylpyrazolin, 3-t-Butylpyrazolin, 3-Chlor-pyrazolin, 3-Brompyrazolin, 3-Ethyl-4-methoxipyrazolin, 1-Acetoxi-3,4-dimethylpyrazolin.

Pyrazolin selbst und substituierte Pyrazoline sind aus technischen Einsatzstoffen leicht zugänglich (siehe z.B. G. Wirsing - J. prakt. Chemie (2) 50, 538).

Als Nitroaromaten können Nitrobenzol oder Nitroderivate anellierter Ringe wie Nitronaphthalin eingesetzt werden. Die Aromaten können ein oder mehrere, insbesondere 1 bis 3 inerte Substituenten tragen. Geeignete Substituenten sind u.a. Alkyl-, Aryl-, Aralkyl-, Amino-, Cyano-, Halogen-, Hydroxi-, Alkoxi-, Carboxyl- oder Sulfogruppen. Es können auch Nitrochinone wie z.B. Nitroanthrachinon oder Nitroderivate von heteroaromatischen Verbindungen wie Pyrrol, Thiophen,. Benzothiophen, Chinolin, Isochinolin, Pyridin oder Imidazol verwendet werden. Diese können ebenso wie die benzoiden Systeme inerte Substituenten tragen. Die Nitroaromaten können außerdem weitere Nitrogruppen enthalten.

Beispielsweise seien folgende Verbindungen aufgeführt: Nitrobenzol, Nitrotoluol, 2,4-Dinitrotoluol, 4-Chlor- oder 4-Bromnitrobenzol, 1,3-Dinitrobenzol, 3-Nitrobenzoesäure, 3-Nitrobenzolsulfonsäure, Nitronaphthalin, Nitroanthrachinon, Nitrochinolin, 2-Nitropyrrol, 2-Nitrothiophen, 4(5)-Nitroimidazol oder 4,5-Dinitroimidazol. Besonders bevorzugt ist Nitrobenzol.

Durch die erfindungsgemäße Verwendung des Nitroaromaten als Dehydriermittel genügt es, katalytische Mengen an Schwefel einzusetzen. So sind bereits Mengen von 0.1 bis 10 Mol%, bezogen auf den Ausgangsstoff Pyrazolin, ausreichend. Größere Mengen, z.B. 10 bis 50 Mol%, sind möglich. bringen aber keine Vorteile.

Die Menge des Nitroaromaten ist nicht besonders kritisch. Im allgemeinen können Molverhältnisse von Pyrazolin:Nitroaromat von 4:1 bis 1:3 gewählt werden. Bei Aromaten mit zwei oder mehr Nitrogruppen kann das Molverhältnis entsprechend reduziert werden. Zweckmäßigerweise verwendet man stöchiometrische Mengen oder einen geringen Überschuß an Nitroaromat, z.B. 1 bis 2 Äquivalente, insbesondere 1 bis 1,5 Äquivalente Nitroaromat pro Mol Pyrazolin.

Die Umsetzung kann in Gegenwart oder Abwesenheit eines Lösungsmittels durchgeführt werden. Als Lösungsmittel kommen beispielsweise in Frage: aliphatische und aromatische Kohlenwasserstoffe, z.B. Toluol, Ethylbenzol, o-, m-, p-Xylol, Methylnaphthalin. Halogenkohlenwasserstoffe, insbesondere Chlorkohlenwasserstoffe. Ether, Alkohole, stickstoffhaltige polare Lösungsmittel wie z.B. Pyridin und Alkylpyridin sowie technische Alkylpyridingemische, Alkylaminopyridine, Chinolin und Alkylchinolin, Morpholin und Alkylmorpholin, alkylsubstituierte Harnstoffe wie Tetramethylharnstoff, Amide wie Dimethylformamid oder Tolylsäurediethylamid sowie Amine, insbesondere aromatische Amine. Auch schwefelhaltige Lösungsmittel wie Dimethylsulfoxid sind möglich. Zweckmäßig verwendet man das Lösungsmittel in einer Menge von 50 bis 10.000 Gew%., vorzugsweise von 100 bis 500 Gew.%, bezogen auf das Pyrazolin.

Eine vorteilhafte Ausführungsform ist die Verwendung des aromatischen Amins, das als Reduktionsprodukt aus dem Nitroaromat entsteht. In vielen Fällen kann auch ohne Lösungsmittel gearbeitet werden.

Die Umsetzung wird besonders vorteilhaft in der Weise durchgeführt, daß man das Pyrazolin zu einem im Reaktionsgefäß vorgelegten Gemisch aus nitroaromatischer Verbindung, Lösungsmittel und katalytischen Mengen Schwefel zudosiert. Der Schwefel liegt meist in gelöster Form vor, er kann aber auch suspendiert oder zusammen mit Aktivkohle verteilt werden.

Die Umsetzung wird üblicherweise bei Umgebungsdruck vorgenommen. In manchen Fällen kann ein leichter Überdruck vorteilhaft sein.

Die Reaktion läuft bei Temperaturen oberhalb von 50 °C ab. Bevorzugt sind Temperaturen von 90 bis 150 °C.

Aus den anfallenden Reaktionsgemischen können die Produkte in an sich bekannter Weise, z.B. durch Destillation oder Extraktion, isoliert werden.

Die nach dem Verfahren der Erfindung herstellbaren Pyrazole sind wertvolle Ausgangsprodukte für die Herstellung von Pflanzenschutzmitteln, Pharmazeutika und Farbstoffen.

Beispiel 1

In einem 250 ml Vierhalskolben wurden 120 g Nitrobenzol und 2 g Schwefel vorgelegt und auf 100 °C erhitzt. Innerhalb von 4 Stunden wurden 50 g Pyrazolin (99 %ig) zugetropft. Anschließend wurde bei 100 °C 2 Stunden nachgerührt und das Reaktionsgemisch (170 g) gaschromatographisch untersucht. Es enthielt 22,8 Gew.% Pyrazol. 3.8 Gew.% Pyrazolin, 9.9 Gew.% Anilin und 57.5 Gew.% Nitrobenzol, daneben noch etwas Wasser und Schwefel. Bei einem Umsatz von 87,4 % Pyrazolin ist die Selektivität 92 % der Theorie.

Beispiel 2

Man verfuhr wie in Beispiel 1, setzte aber innerhalb von 4 Stunden 50 g 4-Methylpyrazolin ein. Man erhielt 170 g Reaktionsgemisch mit 1,8 Gew.% 4-Methylpyrazolin und 25 Gew.% 4-Methylpyrazol. Daraus errechnet sich ein Umsatz von 94 % und eine Selektivität von 92 %.

Beispiel 3

Man verfuhr wie in Beispiel 1, setzte aber innerhalb von 4 Stunden 50 g 5-Methylpyrazolin ein. Man erhielt 170 g Reaktionsgemisch mit 0,2 Gew.% 5-Methylpyrazolin und 27,3 Gew.% 4-Methylpyrazol. Daraus errechnet sich ein Umsatz von 99 % und eine Selektivität von 95 %.

Beispiel 4

Man verfuhr wie in Beispiel 1, setzte aber innerhalb von 4 Stunden 50 g 1-Methylpyrazolin ein. Man erhielt 165 g Reaktionsgemisch mit 24,4 Gew.% 1-Methylpyrazolin und 4,2 Gew.% 1-Methylpyrazol. Daraus errechnet sich ein Umsatz von 19,5 % und eine Selektivität von 73 %.

## Patentansprüche

1. Verfahren zur Herstellung von Pyrazolen durch Dehydrierung von Pyrazolinen bei Temperaturen oberhalb 50 ° C bis zu 150 ° C in Gegenwart von Schwefel, dadurch gekennzeichnet, daß man Pyrazoline mit Nitroaromaten in Gegenwart katalytischer Mengen Schwefel umsetzt.

2. Verfahren nach Anspruch 1, dadurch gekennzeichnet, daß man als Nitroaromaten Nitrobenzol oder substituierte Nitrobenzole verwendet.

3. Verfahren nach Anspruch 1 und 2, dadurch gekennzeichnet, daß man als Nitroaromat Nitronaphthalin oder Nitroanthrachinon verwendet.

4. Verfahren nach den Ansprüchen 1 bis 3, dadurch gekennzeichnet, daß man pro Mol Pyrazolin 0,5 bis 3, insbesondere 1 bis 2 Äquivalente des Nitroaromaten verwendet.

5. Verfahren nach den Ansprüchen 1 bis 4, dadurch gekennzeichnet, daß man 0,1 bis 10 Mol% Schwefel, bezogen auf Pyrazolin, verwendet.

## Claims

1. A process for preparing pyrazoles by dehydrogenation of pyrazolines at above 50 ° C and up to 150 ° C in the presence of sulfur, which comprises reacting pyrazolines with aromatic nitro compounds in the presence of catalytic amounts of sulfur.

2. A process as claimed in claim 1, wherein nitrobenzene or substituted nitrobenzenes are used as aromatic nitro compounds.

3. A process as claimed in claim 1 and 2, wherein nitronaphthalene or nitroanthraquinone is used as aromatic nitro compound.

4. A process as claimed in claims 1 to 3, wherein from 0.5 to 3, in particular from 1 to 2, equivalents of aromatic nitro compound are used per mole of pyrazoline.

5. A process as claimed in claims 1 to 4, wherein from 0.1 to 10 mole % sulfur, based on pyrazoline, are used.

## Revendications

1. Procédé de préparation de pyrazoles par déshydrogénation de pyrazolines à des températures supérieures a 50 ° C jusqu'à 150 ° C, en présence de soufre, caractérisé en ce qu'an fait réagir des

pyrazolines avec des composés aromatiques nitrés en présence de quantités catalytiques de soufre.

**2.** Procédé selon la revendication 1, caractérisé en ce qu'on utilise comme composés aromatiques nitrés, du nitrobenzène ou des nitrobenzènes substitués.

**3.** Procédé selon la revendication 1 ou 2, caractérisé en ce qu'on utilise, comme composé aromatique nitré, du nitronaphtalène ou de la nitroanthraquinone.

**4.** Procédé selon l'une quelconque des revendications 1 à 3, caractérisé en ce qu'on utilise, par mole de pyrazoline, 0,5 à 3, en particulier 1 à 2 équivalents du composé aromatique nitré.

**5.** Procédé selon l'une quelconque des revendications 1 à 4, caractérisé en ce qu'on utilise 0,1 à 10% en moles de soufre, par rapport à la pyrazoline.